# EUROPEAN PATENT SPECIFICATION

(11) **EP 4 389 163 B1**
(45) Date of publication and mention of the grant of the patent: **24.09.2025**
(21) Application number: 22383246.0
(22) Date of filing: 20.12.2022
(51) Int. Cl.: A61L 31/04, A61L 31/16

(54) **IMPROVED SURGICAL MESH FOR HERNIA REPAIR**
VERBESSERTES CHIRURGISCHES NETZ ZUR HERNIENREPARATUR
MAILLE CHIRURGICALE AMÉLIORÉE POUR LA RÉPARATION D'UNE HERNIE

(43) Date of publication of application: 26.06.2024
(73) Proprietor: Fundación Universidad Católica de Valencia San Vicente Mártir, 46001 Valencia (ES)
(72) Inventor: Serrano Aroca, Ángel, 46020 Valencia (ES); Pous Serrano, Salvador, 46007 Valencia (ES)
(74) Representative: Carlos Hernando, Borja

(56) References cited:
- CN-B- 105 050 634
- US-A1- 2016 101 219
- US-B2- 9 801 913
- TUÑÓN-MOLINA ALBERTO ET AL: "Antimicrobial Face Shield: Next Generation of Facial Protective Equipment against SARS-CoV-2 and Multidrug-Resistant Bacteria", INTERNATIONAL JOURNAL OF MOLECULAR SCIENCES, vol. 22, no. 17, 1 September 2021 (2021-09-01), pages 9518, XP093051351, DOI: 10.3390/ijms22179518

## Description

### TECHNICAL FIELD

This invention relates generally to methods and systems for repair of an opening in the body cavity. More specifically, it refers to a surgical mesh for hernia repair in a human or animal body.

### BACKGROUND

One of the most common abdominal wall defects with an indication for surgery is represented by hernias, a protrusion of an organ outside its cavity through an area of low resistance. The hernia may be of different types depending on its location in the human body. It can appear in an inguinal cavity, umbilicus, femoral canal, and epigastrium. Among different types of hernias, the most frequently occurring one is the inguinal (70-75%), followed by femoral (6-17%), umbilical (3-8.5%), incisional (6.2%), and other types such as hiatal, spigelian, or epigastric (8.6%) hernias [1], [2]. Hernia usually makes a visible bulge on the skin, inducing pain and sometimes life-threatening complications in patients.

Hernia repair is one of the most common surgical procedures performed globally. It is estimated that there are over 20 million hernia repair procedures per year worldwide [3]. The number of procedures has been increasing and is predicted to further increase due to several risk factors such as obesity and prior abdominal surgeries [4].

Nowadays, mesh prostheses and no-tension surgical techniques have become a gold standard in treating hernias. In this context, surgical meshes are essential medical devices that support the damaged tissue and its healing. Surgical prostheses for hernia repair aim to strengthen and replace tissue defects, stabilizing the abdominal wall and providing long-term resistance [5].

One of the biomaterials most used for hernia meshes include a wide range of natural and synthetic polymers, with different structures (reticular, laminar, hybrid) and characteristics (pore size, filament distribution). Thus, surgical meshes based mainly on polypropylene (PP), have as their main advantage the high tensile strength (necessary to support intra-abdominal pressure), but also disadvantages related to recurrence, adhesion, and most importantly, infections at the hernia site.

Thus an important risk associated with the use of these prostheses is represented by the associated infections, due to the post-operative complications that affect the patients' quality of life and have a high socio-economic impact [6,7]. In this context, the development of surgical meshes with antimicrobial properties is vital for the prevention of surgical-site infections.

Mesh-related infections could occur weeks or years after surgical intervention and differ from incisional surgical site infections, occurring superficially, within 30 days of the intervention [8]. The data reported for the incidence differs from 1 to 8%, influenced not only by the mesh type but also by patients' co-morbidities (including diabetes and obesity), surgical technique, or strategy for prevention [8,9,10]. The most common microorganisms related to mesh infection are *Staphylococcus aureus* and *Staphylococcus epidermidis,* but infections with *Pseudomonas aeruginosa, Streptococcus pyogenes, Escherichia coli, Klebsiella pneumoniae, Enterococcus faecalis,* or *Candida albicans* have also been reported.

Prosthetic devices are generally made out of materials that do not possess intrinsic antimicrobial activity, thus justifying the infection risk associated with the use of these medical devices.

The strategy for obtaining antimicrobial meshes involve the physical coating or chemical functionalization by introducing different antiseptic/antibacterial agents [12]. The prosthetic devices with prophylactic antimicrobial activity have the surface modified with anti-adhesive substances, metal coatings, or various antimicrobial agents, such as chlorhexidine or rifampicin. These have demonstrated a significant antibacterial activity with reduced bacterial adhesion [11,13], but more antimicrobial meshes coated with antimicrobial compounds are still under research.

The high incidence of the infection due to the said surgical process affect economic aspects, social burden, hospital re-admission, re-operation, hernia recurrence, impaired quality of life and plaintiff litigation [14,15]. In addition, according to the World Health Organization (WHO), antibiotic resistance will be one of the leading causes of death over other important diseases such as cancer by the year 2050 [16]. MRSA is causing global health problems, especially in medical instruments and catheters because *Staphylococcus aureus* is a human pathogen that can easily develop resistance to antibiotics [17,18].

EP2493524B1 describes a medical device comprising: a non-absorbable structure having a surface; a first antimicrobial coating on at least part of the surface, said first antimicrobial coating having a coating surface and comprising a first antimicrobial agent; and a second coating of a polymer comprising a second antimicrobial agent, wherein said second coating is applied over at least part of the coating surface of the first antimicrobial coating, characterized in that the second coating has a discontinuous microstructure comprising discrete droplets of said polymer comprising said second antimicrobial agent. Antimicrobial agents used in the present invention are metal based agents, such as silver and others like triclosan, chlorohexadiene, lauryl acetate and octinedine.

US9457129B2 refers to prosthetic implants such as meshes comprising one or more biodegradable or resorbable polymer coatings comprising antimicrobials selected from rifampin, minocycline, silver/chlorhexidine, vancomycin, a cephalosporin, gentamycin, triclosan and combinations thereof.

US8591531B2 relates to pouches, coverings and the like made from an implantable surgical meshes comprising one or more biodegradable polymer coatings comprising an antimicrobial such as, triclosan, chlorhexidine, rifampin, minocycline (or other tetracycline derivatives), vancomycin, gentamycine, cephalosporins and the like. In preferred embodiments the coatings contain rifampin and another antimicrobial agent, preferably that agent is a tetracycline derivative. Further, the coatings may contain a cephalosporin and another antimicrobial agent. Preferred combinations include rifampin and minocycline, rifampin and gentamycin, and rifampin and minocycline. Further antimicrobials include aztreonam; cefotetan and its disodium salt; loracarbef; cefoxitin and its sodium salt; cefazolin and its sodium salt; cefaclor; ceftibuten and its sodium salt; ceftizoxime; ceftizoxime sodium salt; cefoperazone and its sodium salt; cefuroxime and its sodium salt; cefuroxime axetil; cefprozil; ceftazidime; cefotaxime and its sodium salt; cefadroxil; ceftazidime and its sodium salt; cephalexin; cefamandole nafate; cefepime and its hydrochloride, sulfate, and phosphate salt; cefdinir and its sodium salt; ceftriaxone and its sodium salt; cefixime and its sodium salt; cefpodoxime proxetil; meropenem and its sodium salt; imipenem and its sodium salt; cilastatin and its sodium salt; azithromycin; clarithromycin; dirithromycin; erythromycin and hydrochloride, sulfate, or phosphate salts ethylsuccinate, and stearate forms thereof, clindamycin; clindamycin hydrochloride, sulfate, or phosphate salt; lincomycin and hydrochloride, sulfate, or phosphate salt thereof, tobramycin and its hydrochloride, sulfate, or phosphate salt; streptomycin and its hydrochloride, sulfate, or phosphate salt; vancomycin and its hydrochloride, sulfate, or phosphate salt; neomycin and its hydrochloride, sulfate, or phosphate salt; acetyl sulfisoxazole; colistimethate and its sodium salt; quinupristin; dalfopristin; amoxicillin; ampicillin and its sodium salt; clavulanic acid and its sodium or potassium salt; penicillin G; penicillin G benzathine, or procaine salt; penicillin G sodium or potassium salt; carbenicillin and its disodium or indanyl disodium salt; piperacillin and its sodium salt; ticarcillin and its disodium salt; sulbactam and its sodium salt; moxifloxacin; ciprofloxacin; ofloxacin; levofloxacins; norfloxacin; gatifloxacin; trovafloxacin mesylate; alatrofloxacin mesylate; trimethoprim; sulfamethoxazole; demeclocycline and its hydrochloride, sulfate, or phosphate salt; doxycycline and its hydrochloride, sulfate, or phosphate salt; minocycline and its hydrochloride, sulfate, or phosphate salt; tetracycline and its hydrochloride, sulfate, or phosphate salt; oxytetracycline and its hydrochloride, sulfate, or phosphate salt; chlortetracycline and its hydrochloride, sulfate, or phosphate salt; metronidazole; dapsone; atovaquone; rifabutin; linezolide; polymyxin B and its hydrochloride, sulfate, or phosphate salt; sulfacetamide and its sodium salt; and clarithromycin.

US 2016/101219 A1 discloses a medical product, such as a hernia mesh, with a coating comprising an anti-microbial such as benzalkonium chloride.

One additional concern in the designing of an antimicrobial surgical mesh for hernial repair is to keep fibroblasts proliferative status upon implantation of the surgical mesh so that the healing process is not negatively affected by the incorporation into the mesh of the antimicrobial agents. The antimicrobial surgical mesh needs to be capable of facilitating rapid infiltration of fibroblasts and macrophages into the mesh so that these recruited cells will produce collagen that will form the new connective tissue and anchor the mesh to the injured area.

Meshes loaded with antibiotics may have antibacterial activity against bacteria such as *Escherichia coli* and MRSA, they are not cheap and do not induce cell proliferation [19]. This last property, the induction of cell proliferation, would be important in meshes since they would improve healing after the hernia repair surgery [20].

Consequently, the authors of the present disclosure have reported the need to provide an improved surgical mesh for repair of hernia which in addition to an antimicrobial effect also provides improved fibroblasts proliferative capacity for a rapid and safe tissue regeneration effect at the injured site.

The present invention faced with the above related technical problem provides a more reliable and effective surgical mesh and an implantable medical device comprising such a surgical mesh for repair of amongst others, hernia, which is based on a polymeric substrate coated with benzalkonium chloride.

### SUMMARY

The present disclosure relates to a surgical mesh for repair of an opening in the body cavity. More specifically, the present disclosure is directed to an improved surgical mesh for repair of hernias.

An object of the present disclosure refers to a surgical mesh comprising a mesh substrate having at least a first and/or a second surface, wherein said at least first and/or second surface are covered with a bioabsorbable coating composition comprising benzalkonium chloride (BAK) in an amount of 0,5 to 2.5% w/w with respect to the total weight of the surgical mesh

A further object refers to said surgical mesh, wherein said coating covers at least 10%; 20%, 30%, 40%, 50%, 60%; 70%, 80%, 90%; 99% of the total surface area of said at least first and/or second surfaces of the mesh substrate.

A further object refers to said surgical mesh, optionally comprising in addition a polymer coating onto the surface of the mesh substrate, wherein said polymer coating comprises a biodegradable, resorbable polymer selected from alginate, chitosan, carrageenan, polyhydroxyalkanoate such as polyhydroxybutyrate or poly(3-hydroxybutyrate-co-3-hydroxyvalerate), polylactic acid, polyglycolic acid, polycaprolactone and/or poly lactic-co-glycolic acid (PLGA).

A further object refers to said surgical mesh which is porous, wherein the pore size ranges from 0.1 to 5 mm.

A further object refers to said surgical mesh wherein said mesh substrate is made of a porous non-resorbable synthetic mesh selected from: polypropylene (PP), expanded polytetrafluoroethylene (ePTFE), Polyester (PE), polytetrafluoroetylene, polyvinylidene fluoride (PVDF), PP/PVDF, PP/Poliglecaprone-25, PP/Polidioxanone/oxidated regenerated cellulose, PP/hyaluronic acid+carboxymethylcellulose+polyethylene glycol+polyvinylpyrrolidone, PP/polylactic acid, PE/polylactic acid, polyester (PET) and/or combinations thereof.

A further object refers to said surgical mesh as defined in preceding paragraphs, wherein it further comprises an agent selected from: growth factors, such as: fibroblast growth factor (FGF) and epidermal growth factor (EGF), anti-inflammatory agents, selected from: antibiotics, such as rifampicin, rifampicin-loaded PLGA microspheres, fluoroquinolones (e.g. ofloxacin, ciprofloxacin, levofloxacin), metronidazole, tetracyclines (e.g. minocycline (7-dimethylamino-6-dimethyl-6-deoxytetracycline), minocycline-loaded chitosan nanoparticles, gentamicin, and/or other antimicrobial nanomaterials such as silver, zinc, copper, cobalt, titanium oxide, zinc oxide, copper oxide, cobalt oxide, graphene, graphene oxide, reduced graphene oxide, chlorhexidine, carbon nanotubes, carbon nanofibers, fullerenes, carbon dots, peptides, such as: dermcidin and/or LL-37, other quaternary ammonium compounds such as cetylpyridinium chloride.

A further object refers to said surgical mesh as defined in preceding paragraphs which is in the form of a mesh patch for use over or under the damaged tissue, mesh plug for use inside the hole in the tissue and/or a mesh sheet which can be custom cut and fitted for each uniquely sized injury, for use either over or under the damage tissue.

A further object refers to the surgical mesh as defined in preceding paragraphs for use in the repair of hernias of the type comprising: inguinal, femoral, umbilical, hiatal, incisional, epigastric, spigelian and/or diaphragmatic.

A further object refers to said surgical mesh as defined in preceding paragraphs wherein the mesh substrate is made of polypropylene (PP).

A further object is also an implantable medical device comprising a surgical mesh according to any of the previous paragraphs.

A further object is also a method for producing a surgical mesh as described above for use in the repair of hernias comprising:
a) providing a mesh substrate having at least a first and/or a second surface;
b) applying a benzalkonium chloride (BAK) coating composition onto at least one of the at least first and/or second surfaces of the mesh substrate,
c) drying in an oven at a temperature of from 20 - 50ºC for 12 to 48h, and
d) sterilization under UV radiation for at least 1 h.

wherein said mesh substrate is made of a porous non-resorbable synthetic mesh selected from: polypropylene (PP), expanded polytetrafluoroethylene (ePTFE), Polyester (PE), polytetrafluoroetylene, polyvinylidene fluoride (PVDF), PP/PVDF, PP/Poliglecaprone-25, PP/Polidioxanone/oxidated regenerated cellulose, PP/hyaluronic acid+carboxymethylcellulose+polyethyleneglycol+polyvinylpyrrolidone, PP/polylactic acid, PE/polylactic acid, polyester (PET) and/or combinations thereof and wherein
the coating composition comprises a solution of water in 70% ethanol comprising benzalkonium chloride (BAK) in an amount of from 0,01 - 10% w/v of the solution.

A still further object is a method as defined in the preceding paragraph, which additionally comprises after step a) applying a polymer coating onto the surface of the at least first and/or second mesh substrate, wherein said polymer is a biodegradable, resorbable polymer selected from alginate, chitosan, carrageenan, polyhydroxyalkanoate such as polyhydroxybutyrate or poly(3-hydroxybutyrate-co-3-hydroxyvalerate), polylactic acid, polyglycolic acid, polycaprolactone and/or poly lactic-co-glycolic acid (PLGA) and wherein in step b), the benzalkonium chloride (BAK) coating composition is applied on the surface of the said polymer coating.

A still further object is a method as defined in the preceding paragraphs, wherein applying the benzalkonium chloride (BAK) coating composition of step b) is carried out by plasma spraying, plasma polymerization, vapor deposition or dip-coating.

And finally, it is also an object of the present invention a method as defined in preceding paragraphs, wherein applying the coating composition onto the first and/or second surface of the polymer coated or uncoated mesh substrate is carried out by dip-coating and includes inserting the polymer coated or uncoated mesh substrate into a bath comprising a solution of benzalkonium chloride (BAK) during 1min to 2h at a temperature of 20 - 80ºC.

Unless otherwise defined, all technical and scientific terms used herein have the same meaning as commonly understood by one of ordinary skill in the art to which this disclosure pertains. Although methods and materials similar or equivalent to those described herein can be used in the practice of the present disclosure, suitable methods and materials are described below.

In addition, the materials, methods and examples described herein are illustrative only and are not intended to be limiting. Features and advantages of the disclosure will be apparent from the following detailed description and claims.

### BRIEF DESCRIPTION OF THE DRAWINGS

The foregoing and other objects, aspects, features, and advantages of the present disclosure will become more apparent and better understood by referring to the following description taken in conjunction with the accompanying drawings, in which:
Figure 1 illustrates dip-coating procedure followed to produce antimicrobial PP meshes coated with benzalkonium chloride (BAK).
Figure 2 shows -[4,5-dimethylthiazol-2-yl]-2,5-diphenyl tetrazolium bromide (MTT) cytotoxicity assay of extracts obtained from control, untreated mesh fragment (U Mesh), mesh fragment treated with 70% of ethanol (S Mesh), mesh fragment treated by different BAK disolution (40_BAK, 30_BAK, 20_BAK, 10_BAK, 7.5_BAK, 5_BAK, 2.5_BAK, 1_BAK, 0.5_BAK, 0.1_BAK, 0.05_BAK), positive and negative controls cultured in the presence of fibroblast at 37 °C. The results of the statistical analysis with respect to positive control are indicated in the graph ***p > 0.001; ns, not significant.
Figure 3 shows proliferative activity of mesh fragments with BAK in fibroblast cells stimulated by their exposure to non-cytotoxic concentrations for 72 hours. The results of the statistical analysis with respect to positive control are indicated in the graph ***p > 0.001; ns, not significant.
Figure 4 shows optical microscopy images at two magnifications (x10 and x4) and the macroscopic photographs of the untreated Herniamesh^{®} (U Mesh) and mesh after the treatment with 0.1 and 0.05% BAK (0.1_BAK and 0.05_BAK, respectively) capable of inducing fibroblast proliferation.
Figure 5 shows field emission electron microscopy images at 3 kV and elemental analysis at 10 kV of the untreated Herniamesh^{®} (U Mesh) and mesh after the treatment with 0.1 and 0.05% BAK (0.1_BAK and 0.05_BAK, respectively) capable of inducing fibroblast proliferation. Elemental analysis of the treated and untreated filaments determined by energy-dispersive X-ray spectroscopy. Values of each element express in weight percentage.

### DETAILED DESCRIPTION

The present invention is based, in part, in the findings that coating of a surgical mesh with benzalkonium chloride (BAK) has surprising beneficial effects when used for repair of hernias. These beneficial effects being a combined effect of providing fibroblast proliferative effect while avoiding the occurrence of infections during the healing of said hernial injuries.

Furthermore, the authors of the present invention have found that such beneficial effects are achieved with a surgical mesh coated with a specific amount of BAK, namely from 0.5 to 2.5% w/w with respect to the total weight of the surgical mesh. The surgical mesh described herein may form part of an implantable device used for repair of hernias. Thus, the surgical mesh disclosed herein is a two or three-dimensional substrate made of medical textile having at least a first and a second outer surfaces. The first surface is configured to face a tissue or muscle wall defect, e.g. hernial defect, and the second surface is configured to face away from the defect. Thus, the first and second surfaces are disposed on two opposite faces of the surgical mesh which may form part of the implantable medical device.

In the surgical mesh of the present disclosure, said at least first and/or second surfaces are covered with BAK. The extent of coverage of such at least first and/or second surfaces with BAK therefore comprises at least 10%; 20%, 30%, 40%, 50%, 60%; 70%, 80%, 90%; 99% of the total surface area of said at least first and/or second surfaces of the surgical mesh.

In the surgical mesh of the present disclosure, the substrate is made of medical textiles in the form of a knitted or woven tissue.

The material forming part of the woven or knitted tissue comprise polymer fibers made of polypropylene (PP), expanded polytetrafluoroethylene (ePTFE), Polyester (PE), polytetrafluoroetylene, polyvinylidene fluoride (PVDF), PP/PVDF, PP/Poliglecaprone-25, PP/Polidioxanone/oxidated regenerated cellulose, PP/hyaluronic acid+carboxymethylcellulose+polyethylene glycol+polyvinylpyrrolidone, PP/polylactic acid, PE/polylactic acid, polyester (PET) and/or combinations thereof.

The mesh substrate disclosed herein can be structurally designed to form two or more separate layers arranged on top of each other, where each layer is made of a different polymeric material from the list above.

Surgical mesh according to the present disclosure are also available commercially, from Herniamesh, Klas Medikal, Surgical Mesh, Gore Medical, such as: POLYPROPYLENE (PP) Optilene^{®} Prolene ^{®} Herniamesh^{®}, POLYESTER (PE) Parietex^{®}, POLYTETRAFLUOROETYLENE (cPTFE) Infinit^{®} PTFE mesh, POLYVINYLIDENE FLUORIDE (PVDF) Dynamesh^{®}, PP + TITANIUM TiMesh^{®}, PP WITH SILVER IONS Optilene Siver Mesh^{®},ePTFE (EXPANDED) Gore-Tex^{®} DualMesh^{®},PP/ePTFE Ventralex ^{™} Hernia Patch, PP/PVDF Dynamesh^{®}IPOM, PP/POLIGLECAPRONE-25 Ultrapro^{™}, PP/POLIDIOXANONE/OXIDATED REGENERATED CELULLOSE Proceed^{™} Ventral Patch, PP/HYALURONIC ACID+CARBOXYMETHYLCELLULOSE+ POLYETYLENE GLYCOL Sepramesh ^{®}, PP/ Sepra^{®} hydrogel barrier Ventralex ST^{®} Ventrio ST^{®} Ventralight ST^{®}, POLYGLYCOLIC ACID (PGA) + POLY(TRIMETHYLENE CARBONATE) (PTMC)/PTFE/PGA + PTMC Gore^{®} Synecor, PP/POLYETHYLENE GLYCOL+POLYVINYLPYRROLIDONE Adhesix^{®}, PP/POLILACTIC ACID Parietene^{™} ProGrip^{™}, PE/POLILACTIC ACID Parietex^{™} ProGrip^{™}, PORCINE DERMIS Permacol^{™} Strattice^{™}, PORCINE INTESTINAL SUBMUCOSE Surgisis^{®}, BOVINE PERICARDIUM Veritas^{®}, HUMAN DERMIS Alloderm^{®} FlexHD^{®}AlloMax^{™},BACTERIAL POLYMER MESHES (P4HB) Phasix^{™} and Phasix^{™} ST (Sepra^{®} hydrogel barrier), POLYGLACTIN 910 Vicryl ^{™}, PGA + PTMC Gore^{®} BIO-A^{®}, PGA + PTMC Gore^{®} BIO-A^{®}.

Optical or field emission microscopy shows that the surgical mesh according to the present disclosure maintains a porous structure after having been coated with a BAK composition, having a homogeneous pore size of from 0.1 to 5 mm. More preferably, the surgical mesh has a homogeneous pore structure having a pore size from 0.5 to 3 mm. The surgical mesh as described above is coated with a specific amount of BAK, namely from 0.5 to 7.5% w/w with respect to the total weight of the surgical mesh. Preferably the amount of BAK present on the surface of the surgical mesh is from 0.55 to 2.5%w/w. More preferably, the amount of BAK present on the surface of the surgical mesh is 0.6% w/w; 1% w/w; 1.5% w/w; 1.6% w/w; 2% w/w; or 2.3% w/w, with respect to the total weight of the surgical mesh. BAK is thus physically adsorbed onto the surface of the surgical mesh.

The BAK coating composition used for coating the surgical mesh is an aqueous solution of BAK with concentrations of BAK ranging from 0.01 to 10% w/v of the solution.

Alternatively, the BAK coating composition comprises a solution of water in ethanol, for example, a solution of water in 70% ethanol, where BAK is present in a concentration of from 0.01 to 10% w/v, more preferably, 0.05%, 0.1% and 0.5% w/v.

Suitable BAK compositions are also commercially available from Lab. Montplet, comprising 70% ethanol with 0.1% w/v of BAK, BAK 95.0% pure, BAK 4% aqueous solution, BAK 50% aqueous solution, BAK 10% solution, BAK 50% solution.

Alternatively, the surgical mesh defined above comprises a polymer coating which is a biodegradable, resorbable polymer selected from alginate, chitosan, carrageenan, polyhydroxyalkanoate such as polyhydroxybutyrate or poly(3-hydroxybutyrate-co-3-hydroxyvalerate), polylactic acid, polyglycolic acid, polycaprolactone and/or poly lactic-co-glycolic acid (PLGA). In these embodiments, the BAK coating composition as described above is applied on the surface of the said polymer coating. Thus the polymer coated surgical mesh is also coated with a specific amount of BAK, namely from 0.5 - to 7.5% w/w with respect to the total weight of the surgical mesh. Preferably the amount of BAK present on the surface of the polymer coating of the surgical mesh is from 0.55 to 2.5%w/w. More preferably, the amount of BAK present on the surface of the polymer coating of the surgical mesh is 0.6% w/w; 1% w/w; 1.5% w/w; 1.6% w/w; 2% w/w; or 2.3% w/w, with respect to the total weight of the surgical mesh. BAK is thus physically adsorbed onto the polymer coating surface of the surgical mesh.

Other active ingredients can optionally form part of the surgical mesh, either incorporated into the material forming part of the mesh substrate or into the polymer coating of the surgical mesh. These active ingredients can be selected from: fibroblast growth factor (FGF) and epidermal growth factor (EGF), anti-inflammatory agents, selected from: antibiotics, such as rifampicin, rifampicin-loaded PLGA microspheres, fluoroquinolones (e.g. ofloxacin, ciprofloxacin, levofloxacin), metronidazole, tetracyclines (e.g. minocycline (7-dimethylamino-6-dimethyl-6-deoxytetracycline), minocycline-loaded chitosan nanoparticles, gentamicin, and/or other antimicrobial nanomaterials such as silver, zinc, copper, cobalt, titanium oxide, zinc oxide, copper oxide, cobalt oxide, graphene, graphene oxide, reduced graphene oxide, chlorhexidine, carbon nanotubes, carbon nanofibers, fullerenes, carbon dots, peptides, such as: dermcidin and/or LL-37, other quaternary ammonium compounds such as cetylpyridinium chloride.

The following Table 1 lists several alternatives as disclosed according to the present invention:

| Mesh substrate | BAK (in w/w) | Polymer coating | Other components |
|---|---|---|---|
| PP (Optilene) Optilene^{®} | 0.7%w/w. | Alginate solution | Graphene nanoplatelets |
| PVDF Dynamesh^{®} | 1%w/w. | Chitosan solution | Silver nanoparticles |
| PP/PVDF Dynamesh^{®}IPOM | 2%w/w. | PHBV solution | Fibroblast growth factor |
| PE Parietex^{®} | 2.5%w/w. | PLA solution | Graphene oxide nanosheets |
| ePTFE Gore-Tex^{®} DualMesh^{®} | 0.7%w/w. | None | Rifampicin |
| PP/Poliglecaprone-25 Ultrapro ^{™} | 1%w/w. | Poly(2-hydroxethyl acrylate) solution | Carbon nanofibers |
| PP/PLA Parietene^{™} ProGrip^{™} | 2%w/w. | Polymethyl methacrylate solution | Fibroblast growth factor |
| Polyester/PLA Parietex^{™} ProGrip^{™} | 2.5%w/w. | None | Ofloxacin |
| Silk fiber-based meshes Allergan | 1.5%w/w. | None | Ciprofloxacin |
| Bacterial polymer meshes (P4HB) Phasix^{™} | 2.5%w/w. | None | Levofloxacin |
| PLGA-based meshesVicryl Rapide^{™}(Polyglactin 910) | 2%w/w. | Alginate solution | Carbon nanotubes |
| PLA-based meshes Ethicon | 1.5%w/w. | Chitosane solution | Metronidazole |

A method for producing the surgical mesh herein disclosed for use in the repair of hernias comprises:
a) providing a mesh substrate having a first and a second surface;
b) applying a benzalkonium chloride (BAK) coating composition onto at least one of the first and/or second surfaces of the mesh substrate,
c) drying in an oven at a temperature of from 20 - 50ºC for 12 to 48h, and
d) sterilization under UV radiation for at least 1 h.

wherein said mesh substrate is made of a porous non-resorbable synthetic mesh selected from: polypropylene (PP), expanded polytetrafluoroethylene (ePTFE), Polyester (PE), polytetrafluoroetylene, polyvinylidene fluoride (PVDF), PP/PVDF, PP/Poliglecaprone-25, PP/Polidioxanone/oxidated regenerated cellulose, PP/hyaluronic acid+carboxymethylcellulose+polyethyleneglycol+polyvinylpyrrolidone, PP/polylactic acid, PE/polylactic acid, polyester (PET) and/or combinations thereof and wherein
the BAK coating composition comprises an aqueous solution of BAK comprising BAK in an amount of from 0,01 - 10% w/v of the solution, which preferably is a solution in alcohols, such as methanol, dichloromethane, chloroform, tetrahydrofuran, dimethyl sulfoxide (DMSO) and/or water. More preferably, the coating composition is a solution in water of 70% ethanol comprising BAK in an amount of from 0,01 - 10% w/v of the solution.

Alternatively the said method comprises applying a polymer coating onto the mesh substrate after step a) and before step b), so that in step b), benzalkonium chloride is applied onto at least one of the first and/or second surfaces of the mesh substrate coated with the polymer coating. Said polymer coating is a biodegradable, resorbable polymer selected from alginate, chitosan, carrageenan, polyhydroxyalkanoate such as polyhydroxybutyrate or poly(3-hydroxybutyrate-co-3-hydroxyvalerate), polylactic acid, polyglycolic acid, polycaprolactone and/or poly lactic-co-glycolic acid (PLGA). In these embodiments, the BAK coating composition is applied on the surface of the said polymer coating.

The present disclosure contemplates in the said method of obtaining the surgical mesh, different techniques of applying the coating composition of step b) on the first and/or second surfaces of the polymer coated or uncoated substrate mesh. These techniques are well known to the skilled person and comprise, amongst others: plasma spraying, plasma polymerization, vapor deposition or dip-coating. Plasma treatment is an effective method used to modify the surfaces of biomaterials Among other plasma processes, cold oxygen plasma has been reported as a suitable process to modify, for example, PP fiber surfaces without changing bulk properties. In a preferred embodiment, applying the BAK coating composition onto the first and/or second surface of the surgical mesh is carried out by dip-coating and includes inserting the surgical mesh into a bath comprising a solution of BAK during 1min to 2h at a temperature of 20 - 80ºC.

The surgical mesh according to the present disclosure is anatomically designed and comes in different sizes and shapes that can be adjusted for specific types of hernias. These may include: mesh patch for use over or under the damaged tissue; mesh plug for use inside the hole in the tissue and/or mesh sheet which can be custom cut and fitted for each uniquely sized hernia, either over or under the damaged tissue.

This surgical mesh is intended for use as a permanent or temporary support for organs, injured sites and other tissues during surgery. The primary function of a surgical mesh is to support prolapsed organs either temporarily or permanently. These medical devices are used to provide a physical barrier between types of tissue or extra strength to a physical defect in soft tissue.

Accordingly, the implantable medical device disclosed herein is intended for the treatment of different types of hernias: such as inguinal hernia, femoral hernia, umbilical hernia, hiatal hernia, incisional hernia, epigastric hernia, spigelian hernia and/or diaphragmatic hernia. The surgical mesh supports damaged tissue around hernias as it heals. In further medical uses, the surgical implant disclosed herein is implemented to add as a growth guide for damaged tissue. Preferably, the implantable medical device is strong enough to survive mechanical loads and actions of whichever body area they become a part of. In addition, the presently disclosed surgical mesh facilitates cell proliferation to regenerate tissue, while avoid occurrence of infections in the injured sites. The antibacterial effect of the implantable surgical mesh herein described is due to the action of BAK on the surface of the implantable medical device. The antibacterial effect provided is directed to avoid infections caused by *Staphylococcus aureus and Staphylococcus epidermidis,* but infections with *Pseudomonas aeruginosa, Streptococcus pyogenes, Escherichia coli, Klebsiella pneumoniae, Enterococcus faecalis,* or *Candida albicans* are also prevented. BAK's mechanism of antibacterial action involves altering the cell membrane permeability. The exact mechanism varies with the concentration of BAK solution. Ionic and hydrophobic interaction between the positively charged BAK head and negatively charged bacterial cell membrane leads to loss of coherence in membrane. The bacterial cell loses its physical and ionic stability, resulting in the release of cytoplasmic materials and cellular lysis. In addition, BAK is also effective in inactivating many viruses such as *herpes simplex virus-1, human immunodeficiency virus-1, respiratory syncytial virus,* and *Measles morbillivirus.*

One aspect of the present disclosure is related to developing a surgical mesh that could appropriately integrate with the native tissue, promote its healing and constructive remodeling.

After the surgical mesh has been implanted, an extraordinarily complex series of biological events start and mark the initiation of the healing process.

Fibroblasts are the cells that mediate the wound healing progression. These cells enter the wound site once the acute inflammatory response has receded. The main function of fibroblasts is to synthesize extracellular matrix (EMC) and its collagen components are essential to regenerate the connective tissue.

Fibroblasts play a key role for the success of mesh integration and their optimum amount (density) at the wound is achieved approximately two weeks after surgery.

At first, fibroblasts synthesize an immature, frail collagen type III. A fragile collagen ECM network is produced for around the first 21 days, and then there is a modification in the ratio of collagen type III and I. The collagen type III reduces and the type I, stronger and stable, arises. The mechanical strength increases progressively until 6 months after surgery.

As has been proven in the experimental part of the present disclosure, the surgical mesh disclosed herein have at least 10% increase in fibroblast proliferation effect when compared with the same untreated surgical mesh samples. These surgical mesh samples were characterized as having BAK on the surface of the mesh in an amount of from 0,5 - 2.5% w/w with respect to the total weight of the surgical mesh.

### EXAMPLE

### Materials and methods

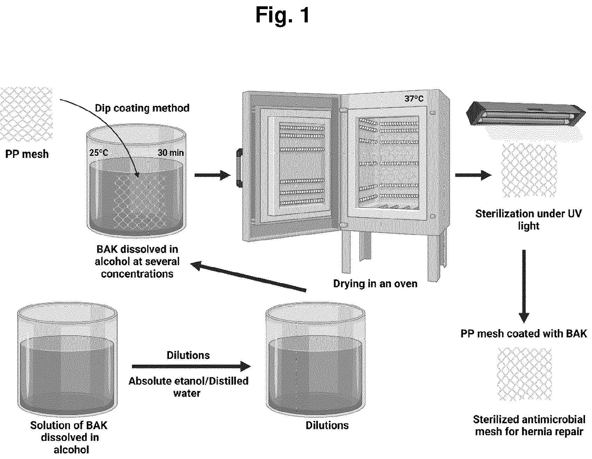

A flat rectangular square macroporous mesh made of non-absorbable polypropylene monofilaments was cut into 1 x 1 cm fragments. The mesh fragments were dried at 37ºC for 24h. A commercial BAK solution at 0.1% w/v (Montplet, Barcelona, Spain) was used as starting solution for providing the definite BA solutions to be used in these experiments. This commercial BAK solution was diluted with an absolute ethanol/distilled water (70/30 v/v) solution at 40%, 30%, 20%, 10%, 7.5%, 5%, 2.5%, 1%, 0.5%, 0.1% and 0.05% v/v. The mesh fragments were treated with these different dilutions by the dip coating method where a coating of BAK was deposited onto the surface by physical adsorption for 30 min at 25ºC (see Figure 1).

For each concentration, six mesh fragments (n=6) were subjected to the same dip-coating treatment. Six mesh fragments were treated with absolute ethanol/distilled water solution (70/30% v/v) without BAK for 30 min at 25 °C as a control (S mesh). Six untreated mesh fragments were produced as untreated control material (U mesh). All treated and untreated mesh fragments were dried at 37 °C for 24 h and sterilized under UV radiation for 1 h per disk side. The BAK used in this study was previously characterized by nuclear magnetic resonance on a BRUKER AVIIIHD 800 MHz (Bruker BioSpin AG, Fälladen, Switzerland) equipped with a 5 mm cryogenic CP-TCI .

### Toxicological Study

Mesh fragments as obtained according to previous paragraph were sterilized under ultraviolet light for 1 h per side. Every biomaterial was placed into a well of a 6-well plate with DMEM (Biowest SAS, France) without Fetal bovine serum (FBS), covering the whole surface area. The ISO-10993 standard recommendations were followed in these cytotoxicity assays. Thus, a volume ratio of 0.1 g/mL was selected according to the ISO-10993 that recommends this rate for irregular porous materials of low density such as textiles.

After incubating the mesh substarte fragments in humidified 5% CO2/95% air ambient for 72 h at 37 °C, the extracts were collected and utilized immediately for the toxicological assay. A L929 mouse fibroblast cell line was used in the cytotoxicity tests. Cell incubation was performed in a mixture of DMEM with 10% FBS, 100 units/mL penicillin (Lonza, Belgium), and 100 mg/mL streptomycin (HyClone, GE Healthcare Life Sciences), at 37 °C and 5% CO2. The effect of the treatment with the mesh fragments on cell viability was measured by the MTT assay. The fibroblast cells were planted at 104 cells/well onto 96-well plate. After incubation for 24 h at 37 °C, the medium in each well was replaced with 100 µL of mesh fragments extracts. The medium was also replaced with 100 µL of the same medium used to produce the mesh fragments extracts (positive control), as well as 100 µL of 1000 µM zinc chloride (≥97.0%, Sigma-Aldrich) solution as negative control because this concentration is highly toxic for the fibroblast [15]. Cell incubation was performed with 5 mg/mL MTT in each well for 3 h. Thus, the formazan crystals were dissolved in 100 µL dimethyl sulfoxide (Sigma-Aldrich) at ambient temperature and the absorbance was read at 550 nm on a microplate reader (Varioskan, Thermo Fisher).

### Proliferation Study

Mesh fragments were sterilized under ultraviolet light for 1 h per side. No toxic concentrations were studied. Every biomaterial was placed into a well of a 6-well plate with DMEM (Biowest SAS, France) without Fetal bovine serum (FBS), covering the whole surface area. The ISO-10993 standard recommendations were followed in these cytotoxicity assays. Thus, a volume ratio of 0.1 g/mL was selected according to the ISO-10993 that recommends this rate for irregular porous materials of low density such as textiles.

After incubating the mesh fragments in humidified 5% CO2/95% air ambient for 72 h at 37 °C, the extracts were collected and utilized immediately for the toxicological assay. A L929 fibroblast cell line was used in this studies. Cell incubation was performed in a mixture of DMEM with 0.5% FBS, 100 units/mL penicillin (Lonza, Belgium), and 100 mg/mL streptomycin (HyClone, GE Healthcare Life Sciences), at 37 °C and 5% CO2. The effect of the treatment with the mesh fragments on proliferation was measured by the MTT assay. The fibroblast cells were planted at 5x103 cells/well onto 96-well plate. After incubation for 24 h at 37 °C, the medium in each well was replaced with 100 µL of mesh fragments extracts. The medium was also replaced with 100 µL of the same medium used to produce the mesh fragments extracts (positive control), as well as 100 µL of 1000 µM zinc chloride (≥97.0%, Sigma-Aldrich) solution as negative control because this concentration is highly toxic for the fibroblast and 100 uL of 15ng/mL of EGF as proliferation control. After incubating the mesh fragments in humidified 5% CO2/95% air ambient for 72 at 37 °C, cell incubation was performed with 5 mg/mL MTT in each well for 3 h. Thus, the formazan crystals were dissolved in 100 µL dimethyl sulfoxide (Sigma-Aldrich) at ambient temperature and the absorbance was read at 550 nm on a microplate reader (Varioskan, Thermo Fisher).

### Mesh characterization

The amount of BAK physically adsorbed to the PP mesh substrates have been determined gravimetrically. Only the meshes that showed biocompatible results for 72 h were characterized. Thus, the treated meshes were dried in vacuo to constant weight and weighted after the dip-coating process to determine the amount of adsorbed BAK.

### Statistical Analysis

Student's t-test was performed for pair comparisons and one-way analysis of variance for multiple value comparisons followed by Tukey's posthoc test (*p > 0.05, ***p > 0.001) on GraphPad Prism 6 software.

### Results

### Toxicological Study

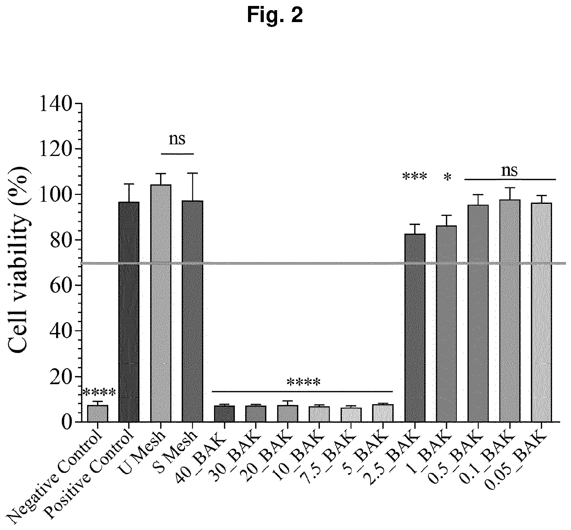

The results of the cytotoxicity tests performed with the BAK extracts in the presence of fibroblast are shown in Figure 2.

Extracts of 1_BAK, 0.5_BAK, 0.1_BAK, 0.05_BAK samples showed no statistically significant differences in cell viability (%) in compared with the positive control. Nevertheless, the 2.5_BAK showed statistically significant differences in cell viability (%). But, the cell viability is greater than 70%, indicating that the sample are not cytotoxic. Finally, the high concentrations of the extracts (40_BAK, 30_BAK, 20_BAK, 10_BAK, 7.5_BAK and 5_BAK) showed statistically significant differences in cell viability (%) and the cell viability is lower than 70%, indicating that the sample are cytotoxic.

### Proliferation Study

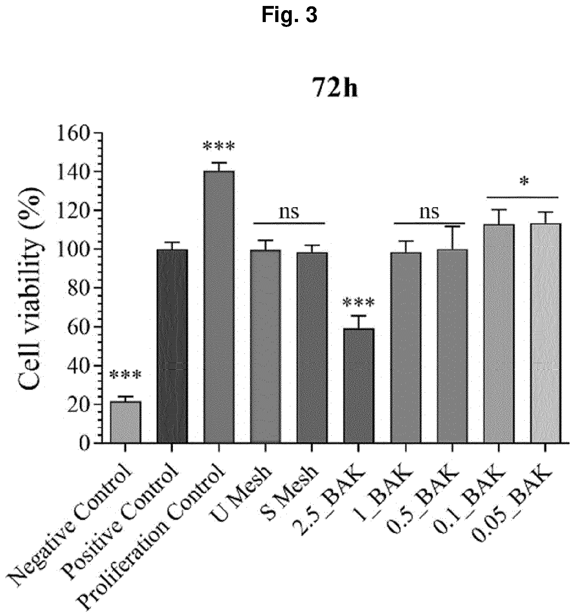

The proliferative activity of mesh fragments with BAK in the fibroblast cell line was studied using non-cytotoxic concentrations (2.5_BAK, 1_BAK, 0.5_BAK, 0.1_BAK and 0.05_BAK) based on the previous results obtained from the cytotoxic assay (Figure 2) to avoid toxic effects by increasing exposure time to 72 h (Figure 3). Thus,

The results at 72h showed that a statistically significant increase in cell growth in the 0.1_BAK and 0.05_BAK concentrations.

### Mesh characterization

The biocompatible amounts of BAK adhered to the PP mesh surface determined gravimetrically are shown in Table 2.

**Table 2. Biocompatible amounts of BAK adhered to the PP mesh surface determined gravimetrically.**

| SAMPLE | BAK (% w/w) |
|---|---|
| 0.1_BAK | 1.98±0.36 |
| 0.05_BAK | 1.05±0.39 |

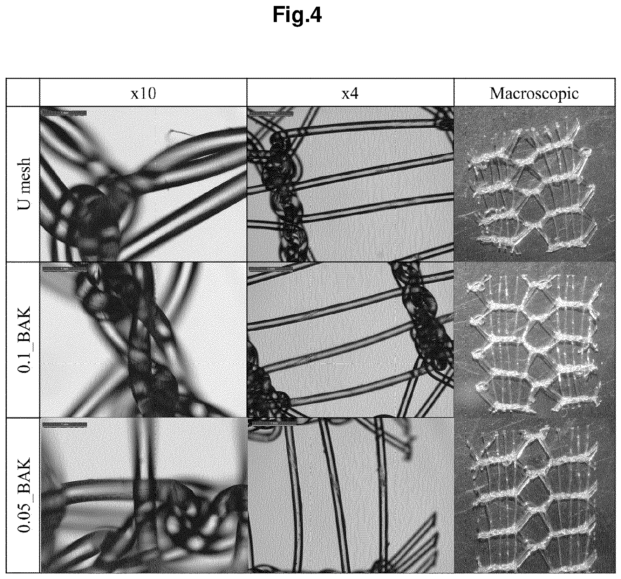

Those physically adsorbed amounts of BAK on the surface of the mesh filaments are so low that the mesh morphology does not change keeping their porous structure, see Figure 4.

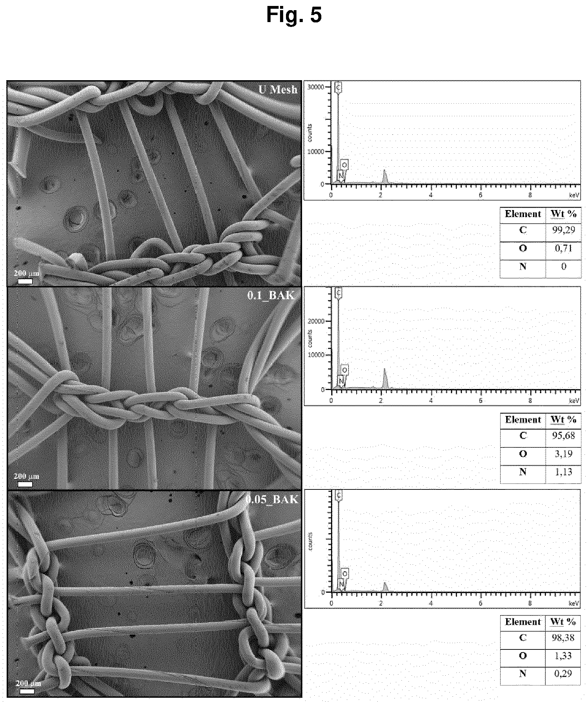

The untreated mesh and the mesh after treatment with 0.1 and 0.05% BAK were also analyzed by field emission electron scanning microscopy, see Figure 5.

### Conclusions

Hernia repair is one of the most common surgical procedures in all the world. Therefore, there is an increase in the use of hernia mesh. The PP implants decrease the recurrence risk and the post-operative pain. Nevertheless, there are many risks associated with the use of hernia mesh such as bacterial infections. We developed a mesh with antimicrobial BAK to prevent this problem. Thus, meshes treated with various concentrations of BAK have been studied. The low concentrations of BAK mesh (treated with 2.5, 1, 0.5, 0.1 and 0.05% v/v dilutions of a commercial BAK solution) demonstrated biocompatibility with fibroblast. In addition, treatment with 0.1 and 0.05% BAK dilutions improve the proliferation on these cells. These surgical mesh samples were characterized as having BAK on the surface of the mesh in an amount of from 0,5 to 2.5% w/w with respect to the total weight of the surgical mesh. These results are promising because these antimicrobial meshes can prevent infections while inducing fibroblast proliferation.

### References:

1.S. Elango, S. Perumalsamy, K. Ramachandran, K. Vadodaria, "Mesh materials and hernia repair", Biomedicine (Taipei), 7 (3) (2017), p. 16, 10.1051/bmdcn/2017070316
2.N. Dabbas, K. Adams, K. Pearson, G.T. Royle, "Frequency of abdominal wall hernias: is classical teaching out of date?", J. R. Soc. Med. Sh. Rep., 2 (1) (2011), pp. 1-6, 10.1258/shorts.2010.010071
3. Kingsnorth A. Treating inguinal hernias: Open mesh Lichtenstein operation is preferred over laparoscopy. BMJ. 2004;328:59-60. doi: 10.1136/bmj.328.7431.59. [PMC free article] [PubMed] [CrossRef] [Google Scholar]
4. Li X., Kruger J.A., Jor J.W., Wong V., Dietz H.P., Nash M.P., Nielsen P.M. Characterizing the ex vivo mechanical properties of synthetic polypropylene surgical mesh. J. Mech. Behav. Biomed. Mater. 2014;37:48-55. doi: 10.1016/j.jmbbm.2014.05.005. [PubMed] [CrossRef] [Google Scholar]
5. Bendavid R., Abrahamson J., Arregui M.E., Flament J.B., Phillips E.H. Abdominal Wall Hernias: Principles and Management. 1st ed. Springer; New York, NY, USA: 2001. [Google Scholar]
6. Usher F.C., Fries J.G., Ochsner J.L., Tuttle L.L. Marlex mesh, a new plastic mesh for replacing tissue defects. II. A new plastic mesh for replacing tissue defects. AMA Arch. Surg. 1959;78:138-145. doi: 10.1001/archsurg.1959.04320010140023. [PubMed] [CrossRef] [Google Scholar]
7. Usher F.C., Hill J.R., Ochsner J.L. Hernia repair with Marlex mesh. A comparison of techniques. Surgery. 1959;46:718-728. [PubMed] [Google Scholar]
8. Brown C.N., Finch J.G. Which mesh for hernia repair? Ann. R. Coll. Surg. Engl. 2010;92:272-278. doi: 10.1308/003588410X12664192076296. [PMC free article] [PubMed] [CrossRef] [Google Scholar]
9. Klinge U., Klosterhalfen B., Schumpelick V. Foreign Body Reaction to Meshes of Used for the Repair of Abdominal Wall Hernias. Eur. J. Surg. 1999;165:665-673. [PubMed] [Google Scholar]
10. Junge K., Klinge U., Prescher A., Giboni P., Niewiera M., Schumpelick V. Elasticity of the anterior abdominal wall and impact for reparation of incisional hernias using mesh implants. Hernia. 2001;5:113-118. doi: 10.1007/s100290100019. [PubMed] [CrossRef] [Google Scholar]
11. Bilsel Y., Abci I. The search for ideal hernia repair; mesh materials and types. Int. J. Surg. 2012;10:317-321. doi: 10.1016/j.ijsu.2012.05.002. [PubMed] [CrossRef] [Google Scholar]
12. Winters J.C., Fitzgerald M.P., Barber M.D. The use of systhetic mesh in female pelvic reconstructive surgery. BJU Int. 2006;98:70-76. doi: 10.1111/j.1464-410X.2006.06309.x. [PubMed] [CrossRef] [Google Scholar]
13. Halm J.A. Ph.D. Thesis. Erasmus University Rotterdam; Rotterdam, The Netherlands: Oct, 2005. Experimental and Clinical Approaches to Hernia Treatment and Prevention. [Google Scholar]
14. T.N. Robinson, J.H. Clarke, J. Schoen, M.D. Walsh, Major mesh-related complications following hernia repair, Surgical Endoscopy And Other Interventional Techniques 2005 19:12. 19 (2005) 1556-1560. https://doi.org/10.1007/S00464-005-0120-Y.
15. R.B. Wilson, Y. Farooque, Risks and Prevention of Surgical Site Infection After Hernia Mesh Repair and the Predictive Utility of ACS-NSQIP, Journal of Gastrointestinal Surgery. (2022) 1-15. https://doi.org/10.1007/S11605-022-05248-6/FIGURES/5.
16. WHO | High levels of antibiotic resistance found worldwide, new data shows, WHO. (2018).
17. S. Lakhundi, K. Zhang, Methicillin-Resistant Staphylococcus aureus: Molecular Characterization, Evolution, and Epidemiology, Clin Microbiol Rev. 31 (2018). https://doi.org/10.1128/CMR.00020-18.
18. D. Chessa, G. Ganau, L. Spiga, A. Bulla, V. Mazzarello, G.V. Campus, S. Rubino, Staphylococcus aureus and staphylococcus epidermidis virulence strains as causative agents of persistent infections in breast implants, PLoS One. 11 (2016). https://doi.org/10.1371/JOURNAL.PONE.0146668.
19. B. Pérez-Köhler, Y. Bayon, J.M. Bellón, Mesh Infection and Hernia Repair: A Review, Https://Home.Liebertpub.Com/Sur. 17 (2016) 124-137. https://doi.org/10.1089/SUR.2015.078.
20. U. Klinge, K. Junge, M. Stumpf, A.P. Öttinger, B. Klosterhalfen, Functional and morphological evaluation of a low-weight, monofilament polypropylene mesh for hernia repair, J Biomed Mater Res. 63 (2002) 129-136. https://doi.org/10.1002/JBM.10119.

## Claims

1. A surgical mesh comprising a mesh substrate having at least a first and/or a second surface, wherein said at least first and/or second surface are covered with a bioabsorbable coating composition comprising benzalkonium chloride (BAK) in an amount of 0,5 to 2.5% w/w with respect to the total weight of the surgical mesh

2. A surgical mesh according to claim 1, wherein said coating covers at least 10%; 20%, 30%, 40%, 50%, 60%; 70%, 80%, 90%; 99% of the total surface area of said at least first and/or second surfaces of the mesh substrate.

3. A surgical mesh according to claim 1 or 2, further comprising a polymer coating onto the surface of the mesh substrate, wherein said polymer coating comprises a biodegradable, resorbable polymer selected from alginate, chitosan, carrageenan, polyhydroxyalkanoate such as polyhydroxybutyrate or poly(3-hydroxybutyrate-co-3-hydroxyvalerate), polylactic acid, polyglycolic acid, polycaprolactone and/or poly lactic-co-glycolic acid (PLGA).

4. A surgical mesh according to any preceding claim which is porous, wherein the pore size ranges from 0.1 to 5 mm.

5. A surgical mesh according to claim 4, wherein said mesh substrate is made of a porous non-resorbable synthetic mesh selected from: polypropylene (PP), expanded polytetrafluoroethylene (ePTFE), Polyester (PE), polytetrafluoroetylene, polyvinylidene fluoride (PVDF), PP/PVDF, PP/Poliglecaprone-25, PP/Polidioxanone/oxidated regenerated cellulose, PP/hyaluronic acid+carboxymethylcellulose+polyethylene glycol+polyvinylpyrrolidone, PP/polylactic acid, PE/polylactic acid, polyester (PET) and/or combinations thereof.

6. A surgical mesh according to any preceding claim, wherein it further comprises an agent selected from: growth factors, such as: fibroblast growth factor (FGF) and epidermal growth factor (EGF), anti-inflammatory agents, selected from: antibiotics, such as rifampicin, rifampicin-loaded PLGA microspheres, fluoroquinolones (e.g. ofloxacin, ciprofloxacin, levofloxacin), metronidazole, tetracyclines (e.g. minocycline (7-dimethylamino-6-dimethyl-6-deoxytetracycline), minocycline-loaded chitosan nanoparticles, gentamicin, and/or other antimicrobial nanomaterials such as silver, zinc, copper, cobalt, titanium oxide, zinc oxide, copper oxide, cobalt oxide, graphene, graphene oxide, reduced graphene oxide, chlorhexidine, carbon nanotubes, carbon nanofibers, fullerenes, carbon dots, peptides, such as: dermcidin and/or LL-37, other quaternary ammonium compounds such as cetylpyridinium chloride.

7. A surgical mesh according to any preceding claim in the form of a mesh patch for use over or under the damaged tissue, mesh plug for use inside the hole in the tissue and/or a mesh sheet which can be custom cut and fitted for each uniquely sized injury, for use either over or under the damage tissue.

8. A surgical mesh according to any preceding claim for use in the repair of hernias of the type comprising: inguinal, femoral, umbilical, hiatal, incisional, epigastric, spigelian and/or diaphragmatic.

9. A surgical mesh according to any preceding claim wherein the mesh substrate is made of polypropylene (PP).

10. An implantable medical device comprising a surgical mesh according to any of claims 1 to 9.

11. A method for producing a surgical mesh according to any of claims 1 to 9 for use in the repair of hernias comprising:
a) providing a mesh substrate having at least a first and/or a second surface;
b) applying a benzalkonium chloride (BAK) coating composition onto at least one of the at least first and/or second surfaces of the mesh substrate,
c) drying in an oven at a temperature of from 20 - 50ºC for 12 to 48h, and
d) sterilization under UV radiation for at least 1 h.
wherein said mesh substrate is made of a porous non-resorbable synthetic mesh selected from: polypropylene (PP), expanded polytetrafluoroethylene (ePTFE), Polyester (PE), polytetrafluoroetylene, polyvinylidene fluoride (PVDF), PP/PVDF, PP/Poliglecaprone-25, PP/Polidioxanone/oxidated regenerated cellulose, PP/hyaluronic acid+carboxymethylcellulose+polyethyleneglycol+polyvinylpyrrolidone, PP/polylactic acid, PE/polylactic acid, polyester (PET) and/or combinations thereof and wherein
the coating composition comprises a solution of water in 70% ethanol comprising benzalkonium chloride (BAK) in an amount of from 0,01 - 10% w/v of the solution.

12. A method according to claim 11, which additionally comprises after step a) applying a polymer coating onto the surface of the at least first and/or second mesh substrate, wherein said polymer is a biodegradable, resorbable polymer selected from alginate, chitosan, carrageenan, polyhydroxyalkanoate such as polyhydroxybutyrate or poly(3-hydroxybutyrate-co-3-hydroxyvalerate), polylactic acid, polyglycolic acid, polycaprolactone and/or poly lactic-co-glycolic acid (PLGA) and wherein in step b), the benzalkonium chloride (BAK) coating composition is applied on the surface of the said polymer coating.

13. A method according to any of claims 11 or 12, wherein applying the benzalkonium chloride (BAK) coating composition of step b) is carried out by plasma spraying, plasma polymerization, vapor deposition or dip-coating.

14. A method according to claim 13, wherein applying the coating composition onto the first and/or second surface of the polymer coated or uncoated mesh substrate is carried out by dip-coating and includes inserting the polymer coated or uncoated mesh substrate into a bath comprising a solution of benzalkonium chloride (BAK) during 1min to 2h at a temperature of 20 - 80ºC.

## Patentansprüche

1. Chirurgisches Netz, das ein Netzsubstrat mit mindestens einer ersten und/oder einer zweiten Oberfläche umfasst, wobei die mindestens erste und/oder zweite Oberfläche mit einer bioresorbierbaren Beschichtungszusammensetzung bedeckt sind, die Benzalkoniumchlorid (BAK) in einer Menge von 0,5 bis 2,5 Gew.-%, bezogen auf das Gesamtgewicht des chirurgischen Netzes, umfasst.

2. Chirurgisches Netz nach Anspruch 1, wobei die Beschichtung mindestens 10 %; 20 %, 30 %, 40 %, 50 %, 60 %; 70 %, 80 %, 90 %; 99 % der Gesamtoberfläche der mindestens ersten und/oder zweiten Oberfläche des Netzsubstrats abdeckt.

3. Chirurgisches Netz nach Anspruch 1 oder 2, das ferner eine Polymerbeschichtung auf der Oberfläche des Netzsubstrats umfasst, wobei die Polymerbeschichtung ein biologisch abbaubares, resorbierbares Polymer umfasst, das aus Alginat, Chitosan, Carrageenan, Polyhydroxyalkanoat wie Polyhydroxybutyrat oder Poly(3-hydroxybutyrat-co-3-hydroxyvalerat), Polymilchsäure, Polyglykolsäure, Polycaprolacton und/oder Polymilchsäure-Co-Glykolsäure (PLGA) ausgewählt ist.

4. Chirurgisches Netz nach einem der vorhergehenden Ansprüche, das porös ist, wobei die Porengröße zwischen 0,1 und 5 mm liegt.

5. Chirurgisches Netz nach Anspruch 4, wobei das Netzsubstrat aus einem porösen, nicht resorbierbaren synthetischen Netz hergestellt ist, das ausgewählt ist aus: Polypropylen (PP), expandiertem Polytetrafluorethylen (ePTFE), Polyester (PE), Polytetrafluorethylen, Polyvinylidenfluorid (PVDF), PP/PVDF, PP/Poliglecapron-25, PP/Polidioxanon/oxidierter regenerierter Cellulose, PP/Hyaluronsäure+Carboxymethylcellulose+Polyethylenglykol+Polyvinylpyrrolidon, PP/Milchsäure, PE/Milchsäure, Polyester (PET) und/oder Kombinationen davon.

6. Chirurgisches Netz nach einem der vorhergehenden Ansprüche, wobei es ferner ein Mittel umfasst, das ausgewählt ist aus: Wachstumsfaktoren wie Fibroblasten-Wachstumsfaktor (FGF) und epidermalem Wachstumsfaktor (EGF), entzündungshemmenden Mitteln, die ausgewählt sind aus: Antibiotika wie Rifampicin, mit Rifampicin beladenen PLGA-Mikrosphären, Fluorchinolonen (z. B. Ofloxacin, Ciprofloxacin, Levofloxacin), Metronidazol, Tetracyclinen (z. B. Minocyclin (7-Dimethylamino-6-dimethyl-6-deoxytetracyclin), mit Minocyclin beladenen Chitosan-Nanopartikeln, Gentamicin und/oder anderen antimikrobiellen Nanomaterialien wie Silber, Zink, Kupfer, Kobalt, Titanoxid, Zinkoxid, Kupferoxid, Kobaltoxid, Graphen, Graphenoxid, reduziertem Graphenoxid, Chlorhexidin, Kohlenstoff-Nanoröhrchen, Kohlenstoff-Nanofasern, Fullerenen, Kohlenstoffpunkten, Peptiden wie: Dermcidin und/oder LL-37, anderen quaternären Ammoniumverbindungen wie Cetylpyridiniumchlorid.

7. Chirurgisches Netz nach einem der vorhergehenden Ansprüche in Form eines Netz-Patches zur Verwendung über oder unter dem geschädigten Gewebe, eines Netzstopfens zur Verwendung im Inneren des Lochs im Gewebe und/oder eines Netzblatts, das für jede spezifische Verletzung individuell zugeschnitten und angepasst werden kann, zur Verwendung entweder über oder unter dem geschädigten Gewebe.

8. Chirurgisches Netz nach einem der vorhergehenden Ansprüche zur Verwendung bei der Reparatur von Leisten-, Schenkel-, Nabel-, Hiatus-, Narben-, epigastrischen, Spieghel- und/oder Zwerchfellhernien.

9. Chirurgisches Netz nach einem der vorhergehenden Ansprüche, wobei das Netzsubstrat aus Polypropylen (PP) besteht.

10. Implantierbare medizinische Vorrichtung, die ein chirurgisches Netz nach einem der Ansprüche 1 bis 9 umfasst.

11. Verfahren zum Herstellen eines chirurgischen Netzes nach einem der Ansprüche 1 bis 9 zur Verwendung bei der Reparatur von Hernien, das Folgendes umfasst:
a) Bereitstellen eines Maschensubstrats mit mindestens einer ersten und/oder einer zweiten Oberfläche;
b) Aufbringen einer Beschichtungszusammensetzung aus Benzalkoniumchlorid (BAK) auf mindestens einer der mindestens ersten und/oder zweiten Oberfläche des Netzsubstrats,
c) Trocknen in einem Ofen bei einer Temperatur von 20 - 50 °C für 12 bis 48 h, und
d) Sterilisieren unter UV-Bestrahlung für mindestens 1 h.
wobei das Netzsubstrat aus einem porösen, nicht resorbierbaren synthetischen Netz hergestellt ist, das ausgewählt ist aus: Polypropylen (PP), expandiertem Polytetrafluorethylen (ePTFE), Polyester (PE), Polytetrafluorethylen, Polyvinylidenfluorid (PVDF), PP/PVDF, PP/Poliglecapron-25, PP/Polidioxanon/oxidierter regenerierter Cellulose, PP/Hyaluronsäure+Carboxymethylcellulose+Polyethylenglykol+Polyvinylpyrrolidon,
PP/Milchsäure, PE/Milchsäure, Polyester (PET) und/oder Kombinationen davon und wobei die Beschichtungszusammensetzung eine Lösung aus Wasser in 70 % Ethanol umfasst, die Benzalkoniumchlorid (BAK) in einer Menge von 0,01 - 10 Gew./Vol.-% der Lösung umfasst.

12. Verfahren nach Anspruch 11, das zusätzlich nach Schritt a) Folgendes umfasst: Aufbringen einer Polymerbeschichtung auf die Oberfläche des mindestens ersten und/oder zweiten Netzsubstrats, wobei das Polymer ein biologisch abbaubares, resorbierbares Polymer ist, das aus Alginat, Chitosan, Carrageenan, Polyhydroxyalkanoat wie Polyhydroxybutyrat oder Poly(3-hydroxybutyrat-co-3-hydroxyvalerat), Polymilchsäure, Polyglykolsäure, Polycaprolacton und/oder Polymilchsäure-Co-Glykolsäure (PLGA) ausgewählt ist und wobei in Schritt b) die Beschichtungszusammensetzung aus Benzalkoniumchlorid (BAK) auf die Oberfläche der Polymerbeschichtung aufgebracht wird.

13. Verfahren nach einem der Ansprüche 11 oder 12, wobei das Aufbringen der Beschichtungszusammensetzung aus Benzalkoniumchlorid (BAK) von Schritt b) durch Plasmaspritzen, Plasmapolymerisation, Aufdampfen oder Tauchbeschichtung erfolgt.

14. Verfahren nach Anspruch 13, wobei das Aufbringen der Beschichtungszusammensetzung auf die erste und/oder zweite Oberfläche des polymerbeschichteten oder unbeschichteten Netzsubstrats durch Tauchbeschichtung erfolgt und das Eintauchen des polymerbeschichteten oder unbeschichteten Netzsubstrats in ein Bad, das eine Lösung aus Benzalkoniumchlorid (BAK) enthält, während 1 min bis 2 h bei einer Temperatur von 20 - 80 °C einschließt.

## Revendications

1. Maille chirurgicale comprenant un substrat de maille ayant au moins une première et/ou une seconde surface, dans laquelle lesdites au moins première et/ou seconde surface sont recouvertes d'une composition de revêtement bioabsorbable comprenant du chlorure de benzalkonium (BAK) en une quantité de 0,5 à 2,5 % p/p par rapport au poids total de la maille chirurgicale.

2. Maille chirurgicale selon la revendication 1, dans laquelle ledit revêtement recouvre au moins 10 % ; 20 %, 30 %, 40 %, 50 %, 60 % ; 70 %, 80 %, 90 % ; 99 % de la surface totale desdites au moins première et/ou seconde surfaces du substrat de maille.

3. Maille chirurgicale selon la revendication 1 ou 2, comprenant en outre un revêtement polymère sur la surface du substrat de maille, dans laquelle ledit revêtement polymère comprend un polymère résorbable, biodégradable choisi parmi l'alginate, le chitosane, le carraghénane, un polyhydroxyalcanoate tel que le polyhydroxybutyrate ou le poly(3-hydroxybutyrate-co-3-hydroxyvalérate), l'acide polylactique, l'acide polyglycolique, la polycaprolactone et/ou le polyacide lactique-coglycolique (PLGA).

4. Maille chirurgicale selon une quelconque revendication précédente qui est poreuse, dans laquelle la taille de pore est comprise dans la plage allant de 0,1 à 5 mm.

5. Maille chirurgicale selon la revendication 4, dans laquelle ledit substrat de maille est constitué d'une maille synthétique poreuse non résorbable choisie parmi : le polypropylène (PP), le polytétrafluoroéthylène expansé (ePTFE), le polyester (PE), le polytétrafluoroéthylène, le fluorure de polyvinylidène (PVDF), PP/PVDF, PP/poliglecaprone-25, PP/polidioxanone/cellulose régénérée oxydée, PP/acide hyaluronique+carboxyméthylcellulose+polyéthylène glycol+polyvinylpyrrolidone, PP/acide polylactique, PE/acide polylactique, le polyester (PET) et/ou des combinaisons de ceux-ci.

6. Maille chirurgicale selon une quelconque revendication précédente, dans laquelle elle comprend en outre un agent choisi parmi : des facteurs de croissance, tels que : le facteur de croissance des fibroblastes (FGF) et le facteur de croissance épidermique (EGF), des agents anti-inflammatoires, choisis parmi : les antibiotiques, tels que la rifampicine, les microsphères de PLGA chargées de rifampicine, les fluoroquinolones (par ex. l'ofloxacine, la ciprofloxacine, la lévofloxacine), le métronidazole, les tétracyclines (par ex. la minocycline (7-diméthylamino-6-diméthyl-6-désoxytétracycline), les nanoparticules de chitosane chargées de minocycline, la gentamicine, et/ou d'autres nanomatériaux antimicrobiens tels que l'argent, le zinc, le cuivre, le cobalt, l'oxyde de titane, l'oxyde de zinc, l'oxyde de cuivre, l'oxyde de cobalt, le graphène, l'oxyde de graphène, l'oxyde de graphène réduit, la chlorhexidine, les nanotubes de carbone, les nanofibres de carbone, les fullerènes, les points de carbone, les peptides, tels que : la dermcidine et/ou LL-37, d'autres composés d'ammonium quaternaire tels que le chlorure de cétylpyridinium.

7. Maille chirurgicale selon une quelconque revendication précédente sous la forme d'un patch en maille destiné à être utilisé sur ou sous le tissu endommagé, d'un bouchon en maille destiné à être utilisé à l'intérieur du trou dans le tissu et/ou d'une feuille en maille qui peut être coupée et ajustée pour chaque lésion de taille unique, destinée à être utilisée sur ou sous le tissu endommagé.

8. Maille chirurgicale selon une quelconque revendication précédente destinée à être utilisée dans la réparation d'hernies du type comprenant : inguinale, fémorale, ombilicale, hiatale, incisionnelle, épigastrique, spigélienne et/ou diaphragmatique.

9. Maille chirurgicale selon une quelconque revendication précédente, dans laquelle le substrat de maille est constitué de polypropylène (PP).

10. Dispositif médical implantable comprenant une maille chirurgicale selon l'une quelconque des revendications 1 à 9.

11. Procédé de fabrication d'une maille chirurgicale selon l'une quelconque des revendications 1 à 9 destinée à être utilisée dans la réparation d'hernies, comprenant :
a) la fourniture d'un substrat de maille ayant au moins une première et/ou une seconde surface ;
b) l'application d'une composition de revêtement de chlorure de benzalkonium (BAK) sur au moins l'une des au moins première et/ou seconde surfaces du substrat de maille,
c) le séchage dans un four à une température de 20 à 50 °C pendant 12 à 48 h, et
d) la stérilisation sous rayonnement UV pendant au moins 1 h.
dans lequel ledit substrat de maille est constitué d'une maille synthétique poreuse non résorbable choisie parmi : le polypropylène (PP), le polytétrafluoroéthylène expansé (ePTFE), le polyester (PE), le polytétrafluoroéthylène, le fluorure de polyvinylidène (PVDF), PP/PVDF, PP/poliglecaprone-25, PP/polidioxanone/cellulose régénérée oxydée, PP/acide hyaluronique+carboxyméthylcellulose+polyéthylèneglycol+polyvinylpyrrolidone,
PP/acide polylactique, PE/acide polylactique, le polyester (PET) et/ou des combinaisons de ceux-ci et dans lequel la composition de revêtement comprend une solution d'eau dans de l'éthanol à 70 % comprenant du chlorure de benzalkonium (BAK) en une quantité de 0,01 à 10 % p/v de la solution.

12. Procédé selon la revendication 11, qui comprend en outre après l'étape a) l'application d'un revêtement polymère sur la surface du au moins premier et/ou du second substrat de maille, dans lequel ledit polymère est résorbable, biodégradable choisi parmi l'alginate, le chitosane, le carraghénane, un polyhydroxyalcanoate tel que le polyhydroxybutyrate ou le poly(3-hydroxybutyrate-co-3-hydroxyvalérate), l'acide polylactique, l'acide polyglycolique, a polycaprolactone et/ou le polyacide lactique-coglycolique (PLGA) et dans lequel à l'étape b), la composition de revêtement de chlorure de benzalkonium (BAK) est appliquée sur la surface dudit revêtement polymère.

13. Procédé selon l'une quelconque des revendications 11 ou 12, dans lequel l'application de la composition de revêtement de chlorure de benzalkonium (BAK) de l'étape b) est réalisée par projection plasma, polymérisation au plasma, dépôt en phase vapeur ou revêtement par immersion.

14. Procédé selon la revendication 13, dans lequel l'application de la composition de revêtement sur la première et/ou la seconde surface du substrat de maille revêtu ou non revêtu de polymère est réalisée par revêtement par immersion et comprend l'insertion du substrat de maille revêtu ou non revêtu de polymère dans un bain comprenant une solution de chlorure de benzalkonium (BAK) pendant 1 min à 2 h à une température de 20 à 80 °C.
